# EUROPÄISCHE PATENTSCHRIFT

(11) **EP 3 315 207 B1**
(45) Veröffentlichungstag und Bekanntmachung des Hinweises auf die Patenterteilung: **02.10.2019**
(21) Anmeldenummer: 16195481.3
(22) Anmeldetag: 25.10.2016
(51) Int. Cl.: B05B 1/26, A61M 15/00, B65D 83/14, A61M 11/00

(54) **SPRÜHKOPF UND VERFAHREN ZU DESSEN HERSTELLUNG**
SPRAY HEAD AND METHOD FOR ITS PRODUCTION
TÊTE DE PULVÉRISATION ET SON PROCÉDÉ DE FABRICATION

(43) Veröffentlichungstag der Anmeldung: 02.05.2018
(73) Patentinhaber: WERRTA GmbH, 14089 Berlin (DE)
(72) Erfinder: Rentsch, Rüdiger, 14089 Berlin (DE)
(74) Vertreter: Ettmayr, Andreas

(56) Entgegenhaltungen:
- EP-A1- 3 097 981
- WO-A2-2009/042212
- US-A1- 2007 163 574

## Beschreibung

Die vorliegende Erfindung betrifft einen Sprühkopf, insbesondere für eine Sprühdose, und ein Verfahren zu dessen Herstellung.

Sprühdosen, oft auch als Spraydosen bezeichnet, sind seit Jahrzehnten in den unterschiedlichsten Anwendungsgebieten bekannt. Sie weisen einen Sprühbehälter auf, in welchem sich ein unter Druck stehendes Treibmittel und das zu versprühende flüssige Sprühgut befindet, sowie einen relativ zum Sprühbehälter beweglich gelagerten Sprühkopf mit mindestens einer Austrittsdüse, aus welcher das Sprühgut austreten kann, wenn durch Bewegen des Sprühkopfes ein Ventil betätigt wird. Je nach Anwendungsgebiet sind dem Fachmann verschiedenste technische Ausführungsformen von Sprühdosen und deren Bestandteilen geläufig.

Die Einheit aus Sprühdose, Treibmittel und Sprühgut wird gemeinhin als Spray bezeichnet. Im medizinischen bzw. pharmazeutischen Bereich finden Sprays insbesondere zur Behandlung von Atemwegserkrankungen Verwendung, beispielsweise von leichten Erkältungserkrankungen mit Beschwerden der oberen Luftwege, aber auch von asthmatischen Beschwerden und anderen Erkrankungen der unteren Luftwege. Zudem können auch Wirkstoffe mittels Sprays verabreicht werden, welche nicht zur lokalen Wirkung in den Luftwegen bestimmt sind, sondern über die Luftwege nur aufgenommen und dann anderweitig im Patientenkörper wirksam werden sollen, beispielsweise Adrenalin zur Behandlung von Schockzuständen.

Die Einsatzmöglichkeiten herkömmlicher Sprühdosen sind begrenzt. Im medizinischen Bereich besteht eine der Grenzen darin, dass sich das Sprühgut für manche Anwendungen mittels herkömmlicher Sprühdosen nicht fein genug zerstäuben lässt, d.h. insbesondere die Erzeugung besonders feiner, lungengängiger Tröpfchen nicht in ausreichendem Maße möglich ist. Einschränkungen beruhen zum einen darauf, dass Behälterdrücke in Sprühbehältern aus Sicherheits- und Kostengründen in der Regel 1,5 MPa(15 bar) kaum überschreiten, zum anderen darauf, dass die Austrittskanäle für das Sprühgut in Sprühkopfdüsen aus fertigungstechnischen Gründen nicht beliebig fein zu wirtschaftlichen Konditionen herstellbar sind.

Ferner eignen sich medizinische Sprays in der Regel nur zur Verabreichung einzelner kurzer Sprühstöße, einer längeren kontinuierlichen Sprühdauer sind durch den Vorrat an Sprühgut und Treibmittel Grenzen gesetzt, zumal die Dosierbarkeit herkömmlicher Sprays eher grob ist, d.h. die Verringerung des austretenden Stroms an Sprühgut zugunsten einer längeren Sprühdauer für den Benutzer in der Regel nicht umsetzbar ist.

Eine Alternative zu medizinischen Sprays stellen technisch aufwendigere Inhalatoren dar, welche mit anderen Energiequellen als komprimiertem Treibmittel arbeiten, beispielsweise elektromechanisch oder mittels Ultraschallzerstäubung. Aufgrund des technischen Aufwands und der damit verbundenen Kosten ist das Anwendungsspektrum derartiger Inhalatoren ebenfalls begrenzt. Insbesondere lassen sie sich nicht wie ein Spray einfach einstecken um bei Bedarf, beispielsweise während sportlicher Betätigung, auf Reisen, oder im normalen Alltagsgeschehen, sofort zur Verfügung zu stehen.

Neben Inhalatoren und herkömmlichen Sprühdosen sind auch Zerstäuberpumpen bekannt, die mittels eines hochgespannten Federsystems im Sprühgut sehr hohe Drücke, teilweise über 200 bar erreichen können. In Kombination mit mikrostrukturierten Düsenkörpern können hiermit sehr feine, lungengängige Tröpfchen erzeugt werden, allerdings nur während kurzer Sprühstöße von maximal etwa 1,5 Sekunden Dauer. Entsprechende Düsenkörper sind zudem aufwendig in der Herstellung. Dabei werden feine Kanäle mittels lithographischer Verfahren aus einem Siliziumdüsenkörper herausgeätzt. Derartige Zerstäuberpumpen und zugehörige Sprühköpfe sind beispielsweise aus EP 1493492 A1 und EP 1386670 A2 bekannt.

Ferner ist bekannt, in einem Sprühkopf zwei oder mehr Düsen so anzuordnen, dass die daraus austretenden Sprühstrahle an an einem von den Austrittsöffungen der Düsen beabstandeten Aufprallort zentral aufeinandertreffen und hierdurch einen feineren Sprühnebel erzeugen, als der jeweilige Einzelstrahl bilden würde. Zentral bedeutet dabei, dass der überwiegende Teil des aus dem einen Austrittskanal als Sprühstrahl austretenden Sprühguts auf den überwiegenden Teil des aus dem anderen Austrittskanal als Sprühstrahl austretenden Sprühguts auftrifft. Eine feine Zerstäubung wird hier insbesondere dadurch erreicht, dass unter Druck ausgestoßenes Sprühgut auf unter Druck ausgestoßenes Sprühgut prallt.

WO 94/07607 A1 beschreibt für die Herstellung eines derartigen, für einen Hochdruck-Inhalationsapparat bestimmten Sprühkopfes, einzelne Schichten mit Laser zu bearbeiten und diese anschließend zusammen zu laminieren. Dieses Verfahren ist überaus aufwendig. In WO 2009/090084 A1 ist die Herstellung eines derartigen, ebenfalls für einen Hochdruck-Inhalationsapparat bestimmten Sprühkopfes, durch Tiefziehen eines Bleches offenbart, in welches zuvor mittels Laserbohrung Öffnungen eingebracht wurden. Mit diesem Herstellungsverfahren lässt sich nur schwer eine ausreichende Fertigungspräzision erzielen. WO 2016/075433 A1 offenbart die Fertigung eines Sprühkopfes als Spritzgussteil aus Kunststoff, wobei die Düsen mittels Laserbohrung erzeugt werden. Auch hier reicht die mögliche Fertigungspräzision kaum dazu aus, eine erwünschte Tröpfchengrößenverteilung durch exakte Auswahl der Düsengeometrie einzustellen.

Insbesondere ist bei den genannte herkömmlichen Sprühköpfen die Oberflächenqualität im Inneren der die Düsen bildenden Austrittskanäle meist unzureichend, so dass der Druckverlust in den Austrittskanälen oft zu hoch ist, und die gewünschte Zerstäubungsleistung nicht erreicht wird. Generell scheitert die Auslegung herkömmlicher Sprühköpfe insbesondere für Sprays mit verhältnismäßig niedrigen Drücken oft daran, dass sich eine gewünschte Tröpfchengrößenverteilung und/oder ein gewünschter Volumenstrom nicht genau genug einstellen lässt.

Angesichts der Einschränkungen denen der Einsatz herkömmlicher Sprühdosen, Inhalatoren und Zerstäuberpumpen unterliegt, liegt der vorliegenden Erfindung die Aufgabe zugrunde, die Nachteile der aus dem Stand der Technik bekannten Sprühköpfe zumindest zu vermindern. Insbesondere liegt der Erfindung auch die Aufgabe zugrunde einen Sprühkopf bereitzustellen, der mit möglichst geringem Kostenaufwand eine möglichst genaue Einstellung des Durchmesserbereichs zulässt, in welchem der Großteil der erzielten Tröpfchendurchmesser liegt, insbesondere zum Verabreichen von medizinischen Wirkstoffen in die Atemwege eines Patienten.

Gemäß einem Aspekt der vorliegenden Erfindung wird diese Aufgabe durch einen Sprühkopf gelöst, welcher mindestens zwei zumindest über einen Teil ihrer Länge in Quarzglas ausgebildete Austrittskanäle aufweist, welche so angeordnet sind, dass aus Austrittsöffnungen jeweils mindestens zweier der Austrittskanäle austretende Sprühstrahle an einem von den Austrittsöffungen beabstandeten jeweiligen Aufprallort zentral aufeinander treffen, wobei die Austrittskanäle jeweils ein als Quotient aus Länge des Austrittskanals und geringstem Durchmesser des Austrittskanals gebildetes Aspektverhältnis von 8 oder kleiner, vorzugsweise von 5 oder kleiner, besonders bevorzugt 3 oder kleiner aufweisen, und der jeweils geringste Durchmesser eines jeden Austrittskanals 50 µm oder kleiner ist.

Durch die Verwendung von Quarzglas lässt sich eine gegenüber dem Stand der Technik erhöhte Fertigungsqualität erzielen. Insbesondere kann die Oberflächenqualität im Inneren der Austrittskanäle und an den Rändern deren Öffnungen verbessert werden, wodurch in der Massenproduktion die erzielbaren Werte für die (vom Druckverlust abhängige) Zerstäubungsleistung und den Sprühgutdurchsatz weniger stark streuen, definierte Sprüheigenschaften somit mit geringeren Abweichungen garantiert werden können.

Vorzugsweise weisen mindestens zwei der Austrittskanäle jeweils einen kleinsten Durchmesser zwischen 5 und 20 µm, besonders bevorzugt zwischen 5 und 15 µm auf. Durch entsprechend kleine Durchmesser lassen sich vorteilhaft niedrige Volumenströme erzielen. Auf diese Weise können mit Sprühdosen handelsüblicher Größe von beispielsweise 100 oder 200 ml Inhalt mehrstündige Inhalationszeiten erreicht werden.

Vorzugsweise weisen die Austrittskanäle jeweils eine Mittelachse auf, welche zur jeweiligen Mittelachse eines jeweils anderen der Austrittskanäle in einem Winkel zwischen 40 und 120 Grad, vorzugsweise zwischen 60 und 100 Grad, besonders bevorzugt zwischen 70 und 90 Grad, steht.

Für die Angaben zur Geometrie des Sprühkopfes gilt folgendes. Als Länge des Austrittskanals wird die geringste Länge von der Berandung der Eintrittsöffnung zur Berandung der Austrittsöffnung des Kanals verstanden. Als Mittelachse des Austrittskanals wird die Gerade verstanden, welche den Flächenschwerpunkt der Eintrittsöffnung mit dem Flächenschwerpunkt der Austrittsöffnung verbindet. Der geringste Durchmesser eines Austrittskanals ist der geringste mögliche Abstand der beiden Schnittpunkte einer die Mittelachse orthogonal schneidenden Linie mit der Berandung des Kanals. Ist der Austrittskanal im wesentlichen zylindrisch jedoch nicht orthogonal zu einer ebenen Oberfläche gebohrt, welche die Berandung der Austrittsöffnung bildet, so ergibt sich eine elliptische Austrittsöffnung, deren kürzere Hauptachse dem geringsten Durchmesser des Austrittskanals entspricht.

Niedrige Aspektverhältnisse gemäß obiger Definition bis hinunter zu 1 und sogar darunter sorgen für einen geringen Energieverlust beim Ausströmen des Sprühguts und eine entsprechend gute Zerstäubung.

Gemäß einer vorteilhaften Weiterbildung der Erfindung sind die vom Rand der jeweiligen Austrittsöffnungen (d.h. der Austrittsöffnungen, deren austretende Sprühstrahle an einem von den Austrittsöffungen beabstandeten jeweiligen Aufprallort zentral aufeinander treffen) aufgespannten Austrittsquerschnittsflächen zueinander geneigt.

Vorteilhafterweise kann der Sprühkopf mindestens zwei Austrittskanäle mit einem ersten jeweiligem geringsten Durchmesser aufweisen, welche so angeordnet sind, dass aus deren Austrittsöffnungen austretende Sprühstrahle an einem von den Austrittsöffungen beabstandeten ersten Aufprallort zentral aufeinander treffen, und mindestens zwei Austrittskanäle mit einem zweiten jeweiligem geringsten Durchmesser, welche so angeordnet sind, dass aus deren Austrittsöffnungen austretende Sprühstrahle an einem von den Austrittsöffungen beabstandeten zweiten Aufprallort zentral aufeinander treffen, wobei der erste jeweilige geringste Durchmesser und der zweite jeweilige geringste Durchmesser, voneinander abweichen, vorzugsweise mindestens im Verhältnis 10:11, besonders bevorzugt mindestens im Verhältnis 2:3. Es werden in dieser Ausführungsform also mindestens zwei Paare oder n-Tupel von Austrittskanälen vorgesehen, deren Sprühstrahle entsprechend an insgesamt zwei oder mehr jeweiligen Aufprallorten zentral aufeinander treffen.

Durch gezielt eingestellte Abweichungen des ersten jeweiligen geringsten Durchmessers vom zweiten jeweiligen geringsten Durchmesser lassen sich gewünschte Tröpfchengrößenverteilungen einstellen. Ebenso können, zusätzlich oder alternativ zu den jeweiligen geringsten Durchmessern, auch die Aspektverhältnisse, vorzugsweise mindestens im Verhältnis 4:5, und/oder die Winkel der Mittelachsen jeweiliger Kanalpaare oder Kanal-n-Tupel, vorzugsweise um mindestens 10°, von denen der übrigen Kanalpaare oder Kanal-n-Tupel abweichen. Insbesondere können durch entsprechend vorgesehene zwei, drei und mehr Paare oder n-Tupel von Austrittskanälen bimodale, trimodale etc. Tröpfchengrößenverteilungen erzielt werden.

Die nachfolgend noch näher beschriebene Fertigung in Quarzglas sorgt für eine genaue Einstellbarkeit des (geringsten) Durchmessers des jeweiligen Austrittskanals und eine insgesamt hohe mögliche Fertigungspräzision. Insbesondere können mit diesem Material besonders gute Oberflächenqualitäten, d.h. geringe Oberflächenrauhigkeiten im Inneren der Austrittskanäle erzielt werden. Entsprechend sind die Austrittskanäle gemäß einer bevorzugten Ausführungsform jeweils vollständig in Quarzglas ausgebildet.

Vorzugsweise sind die Austrittskanäle mittels lokaler Lasereinwirkung auf das Quarzglas und anschließenden Wegätzens von der Lasereinwirkung ausgesetztem Quarzglas gebildet. Das Wegätzen von der Lasereinwirkung ausgesetztem Quarzglas kann beispielsweise unter Verwendung von Flusssäure (HF) oder vorzugsweise Kalilauge (KOH) erfolgen. Das Quarzglas kann auch geeignet dotiert sein, um die Laserbearbeitbarkeit zu verbessern. Ein solches Fertigungsverfahren ist per se unter anderem unter der Bezeichnung SLE (*Selective, Laser-Induced Etching,* selektives laserinduziertes Ätzen) bekannt und in Hermans, M. et al.: Selective, Laser-Induced Etching of Fused Silica at High Scan-Speeds Using KOH, JLMN-Journal of Laser Micro/Nanoengineering Vol. 9, No. 2, 2014 veröffentlicht. Fertigungsmaschinen, mit denen nach dem SLE-Verfahren gefertigt werden kann, sind unter der Bezeichnung LightFab Microscanner kommerziell erhältlich.

Alternativ können die Austrittskanäle vorteilhaft mittels Femtosekundenlaser gebohrt sein. Die Herstellung mittels lokaler Lasereinwirkung auf das Quarzglas und anschließenden Wegätzens von der Lasereinwirkung ausgesetztem Quarzglas gestattet jedoch in der Regel höhere Fertigungsqualität.

Gemäß einer weiteren vorteilhaften Ausführungsform können die Austrittskanäle jeweils in einem Abschnitt einer gezogenen Glaskapillare ausgebildet sein, wobei mindestens eine Glaskapillare mit einem Innendurchmesser gezogen wird, der dem geringsten Durchmesser eines Austrittskanals entspricht, die mindestens eine Glaskapillare so geschnitten wird, dass für jeden Austrittskanal ein diesen bildender Abschnitt entsteht, und die Abschnitte so zueinander angeordnet werden, dass die durch die Abschnitte gebildeten Austrittskanäle einander zugeneigt sind. Die Glaskapillare wird somit in Scheiben geschnitten, wobei aus einer Scheibe jeweils ein Austrittskanal gebildet wird. Mit dem Pikosekundenlaser wird überschüssige Material aus dem Düsenkanal abgetragen bis das richtige Aspektverhältnis erreicht ist. Anschließend werden zwei fertige Einlochdüsen in einen geeigneten Düsengehäusekörper eingesetzt.

Vorteilhafterweise kann der Sprühkopf eine Sprühkopfkulisse aus einem Kunststoff, beispielsweise Polyethylen, aufweisen, in den der oder die die Austrittskanäle enthaltenden Düsenkörper aus Quarzglas eingefügt sind, was dazu beiträgt, die Fertigungskosten gering zu halten. Jedoch können auch andere, beispielsweise metallische, Werkstoffe verwendet für eine Sprühkopfkulisse verwendet werden.

Besonders kostengünstig kann die Sprühkopfkulisse als Spritzgussteil ausgeführt sein.

Je nachdem, wie breit oder schmal die Auffächerung des beim Sprühvorgang erzeugten Aerosol-Strahls erwünscht ist, können die Austrittskanäle an ihren Austrittsöffnungen vorteilhafterweise in einen weiteren oder engeren Sprühkanal münden.

Gemäß einer besonders vorteilhaften Ausführungsform weist der Sprühkopf Adapterverbindungsmittel für einen Patientenluftwegeadapter auf, wobei der Sprühkopf mittels der Adapterverbindungsmittel werkzeugfrei, beispielsweise mittels einer Steck- oder Schraubverbindung, so mit dem Patientenluftwegeadapter verbindbar ist, dass aus den Austrittsöffnungen ausgetretenes Sprühgut in den Patientenluftwegeadapter eintreten kann. Ein solcher Patientenluftwegeadapter als in eine Patientennase einführbarer Nasenadapter, als Mundstück, oder als einen Patientenmund und/oder eine Patientennase abdeckender Maskenadapter ausgebildet sein.

Ebenfalls vorteilhaft kann der Sprühkopf mit einem Patientenluftwegeadapter integral geformt oder verbunden sein, der wiederum als in eine Patientennase einführbarer Nasenadapter, als einen Patientenmund und/oder eine Patientennase abdeckender Maskenadapter oder als mit einem Patientenmund umschließbarer Adapter ausgebildet ist.

Vorteilhafterweise kann der Patientenluftwegeadapter aus einem geeigneten Kunststoff wie etwa PE oder PP, beispielsweise als Spritzgussteil, ausgeführt sein.

Gemäß einer weiteren vorteilhaften Ausführungsform kann der Sprühkopf mit einer Adapterhülle zum Einführen eines Sprühbehälters verbunden oder integral ausgebildet sein, wobei über die Behälterverbindungsmittel eine werkzeugfrei schließbare und werkzeugfrei lösbare Verbindung mit dem Sprühbehälter herstellbar ist. So lässt sich der Sprühkopf mit geeigneten Einweg-Sprühbehältern nutzen und resourcenschonend mehrfach verwenden.

Gemäß einem weiteren Aspekt betrifft die Erfindung eine Sprühdose, welche einen Sprühkopf der obigen Art und den Sprühbehälter aufweist. Vorzugsweise übersteigt der Druck im Sprühbehälter 3 MPa (30 bar) nicht, bevorzugt übersteigt er 1.5 MPa (15 bar), besonders bevorzugt 1 MPa (10 bar) nicht. Auch im Bereich von Behälterdrücken von 0,3 bis 10 MPa (3 bis 10 bar) erreicht die erfindungsgemäße Sprühdose noch eine wesentlich feinere Tröpfchengrößen als herkömmliche Sprühdosen mit Treibmittel.

Für den besonders bevorzugten Anwendungsbereich der erfindungsgemäßen Sprühdose ist der Sprühbehälter mit zumindest einem medizinischen Wirkstoff, vorzugsweise Sole, und einem Treibmittel befüllt. Insbesondere bei der Verwendung von Sole kommt der erfindungsgemäße Vorteil zum Tragen, dass sich eine breite Tröpfchengrößenverteilung erzielen lässt. Sole kann so vorteilhafterweise an die oberen und unteren Luftwege gleichermaßen verabreicht werden. Insbesondere Sole eignet sich auch deshalb für eine gleichzeitige Verabreichung an die oberen und unteren Luftwege, da es sich hierbei um einen weitestgehend nebenwirkungsfreien Stoff handelt. Wenn bei einer der Behandlung eines Patienten vorausgehenden Diagnose oder Selbstdiagnose nicht eindeutig klar ist, in welchem Bereich der Luftwege die Solegabe benötigt wird, kann mit Tröpfchen unterschiedlicher Größe ein vorsorglich auf unterschiedliche Bereiche der Luftwege zugleich einwirkender Therapieansatz verfolgt werden.

Entsprechend den obigen Ausführungen betrifft die vorliegende Erfindung gemäß einem weiteren Aspekt auch ein Verfahren zum Herstellen eines Sprühkopfes, wobei zwei einander zugeneigte Austrittskanäle mit einem jeweiligen geringsten Durchmesser von 50 µm oder kleiner zumindest teilweise in Quarzglas so gebildet werden, dass die Austrittskanäle jeweils ein als Quotient aus Länge des Austrittskanals und geringstem Durchmesser des Austrittskanals gebildetes Aspektverhältnis von 8 oder kleiner, vorzugsweise von 5 oder kleiner, besonders bevorzugt 3 oder kleiner aufweisen.

Auch wenn der erfindungsgemäße Sprühkopf hauptsächlich für medizinische Anwendungen beschrieben wird, ist die Erfindung darauf nicht beschränkt. Vielmehr lässt sich der erfindungsgemäße Sprühkopf in einer Vielzahl von technischen Anwendungen einsetzen, in welchen eine feine Zerstäubung bei niedrigen Volumenströmen und begrenzten verfügbaren Druckdifferenzen gewünscht wird, beispielsweise für die Desinfektion von Klimaanlagen oder mit Druckluft betriebene Vernebler für zur Atemluft- oder Hautoberflächenbefeuchtung in Kliniken. Auch in kosmetische Anwendungen ist eine besonders feine Zerstäubung oft wünschenswert. Hier lassen sich Sprays mit erfindungsgemäßen Sprühköpfen ebenfalls produktwertsteigernd einsetzen.

Die Erfindung wird nachfolgend in beispielhafter Weise anhand der beigefügten schematischen Zeichnung näher erläutert. Die Zeichnungen sind nicht maßstabsgetreu; insbesondere entsprechen Verhältnisse der einzelnen Abmessungen zueinander aus Gründen der Anschaulichkeit teilweise nicht den Abmessungsverhältnissen in tatsächlichen technischen Umsetzungen. Es werden mehrere bevorzugte Ausführungsbeispiele beschrieben, auf welche die Erfindung jedoch nicht beschränkt ist. Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar und im Umfang der Ansprüche, sind einzelne Merkmale der beschriebenen Ausführungsformen austauschbar oder miteinander sowie mit per se aus dem Stand der Technik bekannten Merkmalen kombinierbar.

Es zeigt
- Fig. 1: die Querschnittsdarstellung einer erfindungsgemäßen Sprühdose mit einem erfindungsgemäßen, mit einem Nasenadapter integral geformten Sprühkopf und einem Sprühbehälter,
- Fig. 2: den aus Quarzglas gefertigten Düsenkörper des Sprühkopfes aus Fig. 1, ebenfalls in Querschnittsdarstellung.
- Fig. 3: die Querschnittsdarstellung eines erfindungsgemäßen, mit einem Mundstück integral geformten Sprühkopfs mit einem Düsenkörper wie dem in Fig. 2 dargestellten,
- Fig. 4: einen erfindungsgemäßen Sprühkopf, wiederum in Querschnittsdarstellung, mit aufgeschraubter Mund-/Nasenmaske und einem Düsenkörper wie dem in Fig. 2 dargestellten,
- Fig. 5a: den aus Quarzglas gefertigten Düsenkörper aus Fig. 2 in der Draufsicht von unten, wobei die Schnittebene der Fig. 2 als durchbrochene Linie A-A' angedeutet ist,
- Fig. 5b: den Düsenkörper aus Fig. 2 abermals in Querschnittsdarstellung zum Vergleich mit der zugehörigen Draufsicht (von unten) in Fig. 5a,
- Fig. 6a: einen aus Quarzglas gefertigten Düsenkörper in einer Draufsicht ähnlich Fig. 5a, wobei der Düsenkörper drei Paare von Austrittskanälen mit von Paar zu Paar abweichendem Kanaldurchmesser aufweist, und die jeweilige Schnittebene der Figuren 6b, 6c und 6d als durchbrochene Linien B-B', C-C' bzw. D-D' angedeutet ist,
- Fig. 6b: den Düsenkörper aus Fig. 6a in der Querschnittsdarstellung für die Schnittebene B-B',
- Fig. 6c: den Düsenkörper aus Fig. 6a in der Querschnittsdarstellung für die Schnittebene C-C',
- Fig. 6d: den Düsenkörper aus Fig. 6a in der Querschnittsdarstellung für die Schnittebene D-D',
- Fig. 7: die Querschnittsdarstellung eines erfindungsgemäßen, mit einem Mundstück integral geformten Sprühkopfs ähnlich Fig. 3 jedoch mit zwei aus Quarzglaskapillaren gefertigten Düsenkörpern,
- Fig. 8: eine vergrößerte Darstellung der Anordnung der Düsenkörper aus Fig. 7,
- Fig. 9a-c: einen Düsenkörper aus Figuren 7 und 8 in drei verschiedenen Fertigungsschritten,
- Fig. 10a-c: Düsenkörper in drei verschiedenen Fertigungsschritten ähnlich Fig. 9a-c jedoch aus einer schräg geschnittenen Kapillare,
- Fig. 11: eine erfindungsgemäße Sprühdose mit seitlich austragendem Sprühkopf im Querschnitt, und
- Fig. 12: eine erfindungsgemäße Sprühdose ähnlich Fig. 11 jedoch mit Gewindestutzen zum Aufsetzen eines Adapters.

Einander entsprechende Elemente sind in den Zeichnungsfiguren jeweils mit den gleichen Bezugszeichen bezeichnet.

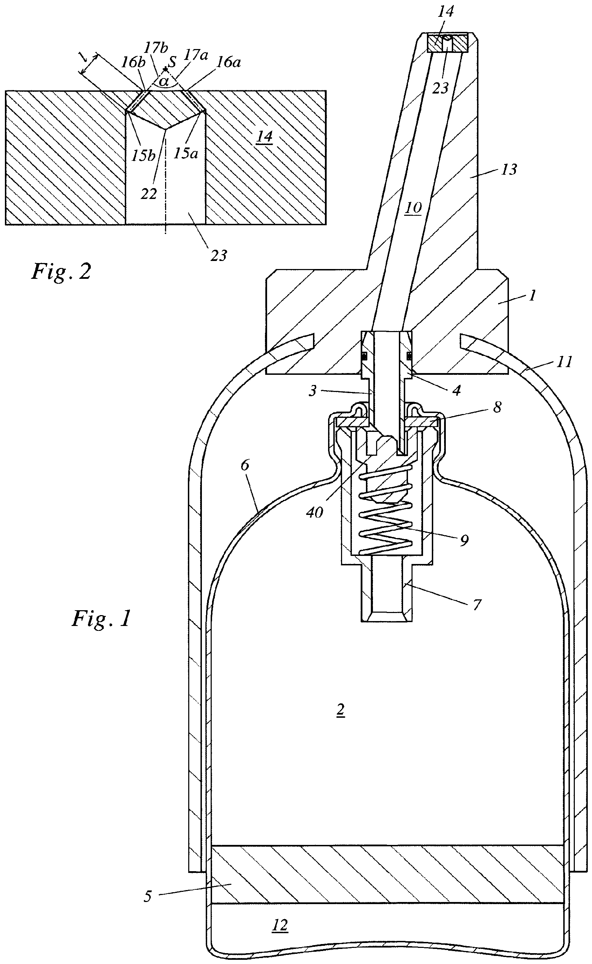

Der wiederverwendbare Sprühkopf 1 in Fig. 1 ist auf den Kopf 4 des Austrittsrohrs 3 des Sprühbehälters 2 aufgesetzt und von diesem werkstofffrei abziehbar, um den Austausch des Sprühbehälters 2 zu ermöglichen. Alternativ ist auch eine Ausführung als Einwegprodukt möglich.

Der Sprühbehälter 2 ist als Zweikammerbehälter ausgeführt, in welchem ein verschieblicher Kolben 5 das im unteren Bereich 12 des Sprühbehälters 2 vorhandene komprimierte Treibmittel, beispielsweise Stickstoff, vom Sprühgut, beispielsweise Sole, trennt. Alternativ lässt sich die Erfindung auch mit einem Einkammerbehälter als Sprühbehälter 2 ausführen, bei welchem Treibmittel und Sprühgut vermischt sind. Als Treibmittel eignen sich die aus herkömmlichen Sprays bekannten Treibmittel. Die Behälterwand 6 des Sprühbehälters 2 ist aus herkömmlichem Behältermaterial wie beispielsweise Aluminium oder Weißblech gefertigt.

Das Ventil des Sprühbehälters 2 ähnelt den Ventilen herkömmlicher Sprühdosen und weist ein Ventilgehäuse 7 auf, welches über den aus einem Elasten wie Kautschuk- oder Silikongummi bestehenden Dichtring 8 abgedichtet ist. Die ins Ventilgehäuse eingesetzte Feder 9 drückt die Verschlusskapsel 40 gegen den Dichtring 8. Durch Zusammendrücken von Sprühkopf 1 und und Sprühbehälter 2 relativ zu einander schiebt das unten angeschrägte Austrittsrohr 3 die Verschlusskapsel nach unten, so dass Sprühgut durch Ventilgehäuse 7 und Austrittsrohr 3 in den Zuführungskanal 10 des Sprühkopfs 1 eintreten kann. Um die Handhabung zu erleichtern und Verkannten beim Zusammendrücken zu vermeiden, ist der Sprühbehälter 2 in einer mit dem Sprühkopf 1 fest verbundenen Adapterhülle 11 geführt.

Der Sprühkopfkulisse 13 besteht aus thermoplastischem oder duroplastischen Kunststoff, z.B. Polyethylen, Polypropylen oder Polycarbonat, und ist als in ein Nasenloch einführbarer Nasenadapter ausgeformt. Eingesetzt in die Sprühkopfkulisse 13 ist der Düsenkörper 14, welcher in Fig. 2 vergrößert dargestellt ist.

Mittels selektiver Laserbelichtung und anschließendem Ausätzen der belichteten Bereiche (Selektives laserinduziertes Ätzen) sind in dem zylindrischen Quarzglas-Düsenkörper 14 zwei im wesentlichen zylindrische Austrittskanäle 15a, 15b mit Austrittsöffnungen 16a, 16b gebildet. Die im Düsenkörper 14 ausgeformte Fortsetzung 23 des Zuführungskanals 10 ist durch Materialabtrag mittels Picosekundenlaser hergestellt. Der sich quer durch den zylindrischen Querschnitt der Fortsetzung 23 des Zuführungskanals 10 erstreckende Steg 22 dient der Stabilität insbesondere bei hohen Drücken.

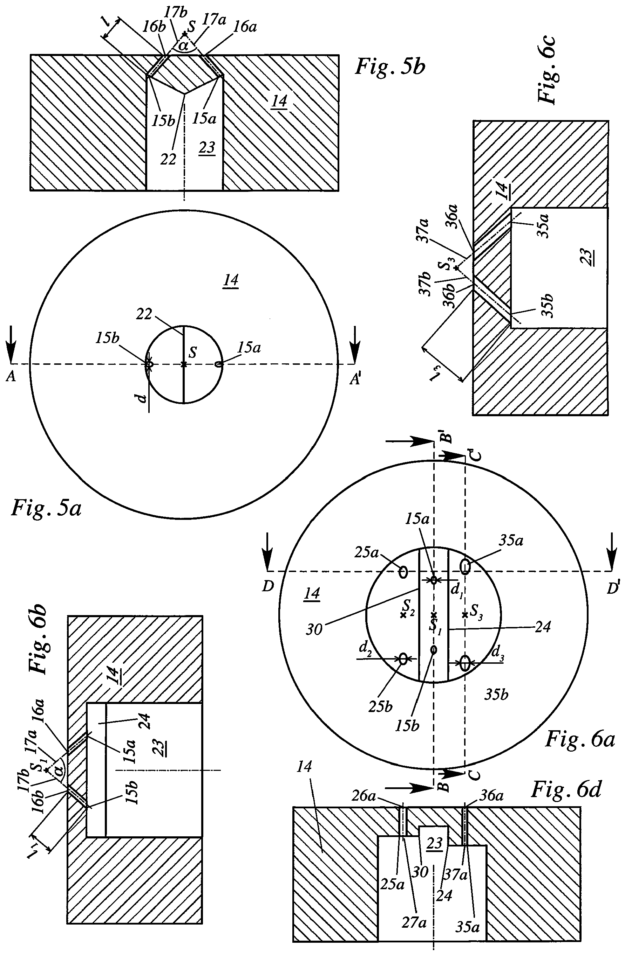

Der Winkel α zwischen den sich im Punkt S schneidenden Mittelachsen 17a, 17b der Austrittskanäle 15a, 15b beträgt etwa 80°. Das Aspektverhältnis zwischen der Länge 1 der Austrittskanäle 15a, 15b (entlang der jeweils kürzesten Mantellinie des zylindrischen Mantels der Austrittskanäle 15a, 15b) und deren Zylinderdurchmesser beträgt ca. 5:1. In Figuren 5a und 5b sind die geometrischen Verhältnisse nochmals verdeutlicht. Fig. 5a ist dabei die Draufsicht von unten in Fig. 5b. Die Schnittebene der Fig. 5b ist in Fig. 5a als durchbrochene Linie A-A' angedeutet.

Durch geeignete Wahl des Aspektverhältnisses, des treibenden Druckgefälles, des Strahl-Prallwinkels α sowie des Durchmessers d (und entsprechender Länge 1) der Austrittskanäle 15a, 15b lassen sich die Tröpfchengrößenverteilung und der Volumenstrom einstellen.

Für wässrige Solelösung mit einem Salzgehalt von 0,9 Massenprozent als Sprühgut haben sich (bei der gegebenen Messgenauigkeit) gemäß Arbeiten des Anmelders bei einer treibenden Druckdifferenz von 0,3 MPa (3 bar) und einem Strahl-Prallwinkel α von 80° Tröpfchengrößenverteilungen mit folgenden Sauterdurchmessern (d₃₂) sowie die folgenden Volumenströme Q ergeben:

| | |
|---|---|
| Q=0,006 ml/s und d₃₂ = 50,9 µm | bei d = 20 µm und l = 100 µm |
| Q=0,003 ml/s und d₃₂ = 50,0 µm | bei d = 15 µm und l = 75 µm |
| Q=0,001 ml/s und d₃₂ = 51,0 µm | bei d = 10 µm und l = 50 µm |
| Q=0,001 ml/s und d₃₂ = 52,8 µm | bei d = 8 µm und l = 40 µm |

Bei einer treibenden Druckdifferenz von 0,5 Mpa (5 bar) und sonst gleichen Parametern ergab sich:

| | |
|---|---|
| Q=0,010 ml/s und d₃₂ = 32,1 µm | bei d = 20 µm und l = 100 µm |
| Q=0,005 ml/s und d₃₂ = 30,7 µm | bei d = 15 µm und l = 75 µm |
| Q=0,002 ml/s und d₃₂ = 30,0 µm | bei d = 10 µm und l = 50 µm |
| Q=0,001 ml/s und d₃₂ = 30,2 µm | bei d = 8 µm und l = 40 µm |

Bei einer treibenden Druckdifferenz von 1 MPa (10 bar) und sonst gleichen Parametern ergab sich:

| | |
|---|---|
| Q=0,016 ml/s und d₃₂ = 19,3 µm | bei d = 20 µm und l = 100 µm |
| Q=0,008 ml/s und d₃₂ = 18,0 µm | bei d = 15 µm und l = 75 µm |
| Q=0,003 ml/s und d₃₂ = 16,9 µm | bei d = 10 µm und l = 50 µm |
| Q=0,002 ml/s und d₃₂ = 16,5 µm | bei d = 8 µm und l = 40 µm |

Bei einer treibenden Druckdifferenz von 1,2 MPa (12 bar) und sonst gleichen Parametern ergab sich:

| | |
|---|---|
| Q= 0,018 ml/s und d₃₂ = 17,1 µm | bei d = 20 µm und l = 100 µm |
| Q= 0,009 ml/s und d₃₂ = 15,9 µm | bei d = 15 µm und l = 75 µm |
| Q= 0,004 ml/s und d₃₂ = 14,7 µm | bei d = 10 µm und l = 50 µm |
| Q=0,002 ml/s und d₃₂ = 14,4 µm | bei d = 8 µm und l = 40 µm |

Bei einer treibenden Druckdifferenz von 1.5 MPa (15 bar) und sonst gleichen Parametern ergab sich:

| | |
|---|---|
| Q=0,020 ml/s und d₃₂ = 14,8 µm | bei d = 20 µm und l = 100 µm |
| Q=0,011 ml/s und d₃₂ = 13,7 µm | bei d = 15 µm und l = 75 µm |
| Q=0,004 ml/s und d₃₂ = 12,6 µm | bei d = 10 µm und l = 50 µm |
| Q=0,003 ml/s und d₃₂ = 12,2 µm | bei d = 8 µm und l = 40 µm |

Bei einer treibenden Druckdifferenz von 2 MPa (20 bar) und sonst gleichen Parametern ergab sich:

| | |
|---|---|
| Q=0,024 ml/s und d₃₂ = 12,3 µm | bei d = 20 µm und l = 100 µm |
| Q=0,013 ml/s und d₃₂ = 11,3 µm | bei d = 15 µm und l = 75 µm |
| Q=0,005 ml/s und d₃₂ = 10,3 µm | bei d = 10 µm und l = 50 µm |
| Q=0,003 ml/s und d₃₂ = 9,9 µm | bei d = 8 µm und l = 40 µm |

Wie sich aus den angegebenen Volumenströmen ersehen lässt, können mit Sprühdosen handelsüblicher Größe von beispielsweise 100 oder 200 ml Inhalt mehrstündige Inhalationszeiten erreicht werden.

Erkennbar ist, dass die Abhängigkeit der Tröpfchengröße vom Kanaldurchmesser mit höherer treibender Druckdifferenz steigt.

Durch Variieren des Aspektverhältnisses und auch des Aufprallwinkels der Sprühstrahle lässt sich die Tröpchengrößenverteilung ebenfalls beeinflussen, wobei für geringere Aspektverhältnisse und somit hören Energieeintrag beim Auftreffen der Sprühstrahle aufeinander meist ein größerer Feinanteil der Tröpfchengrößenverteilung erzielt werden kann.

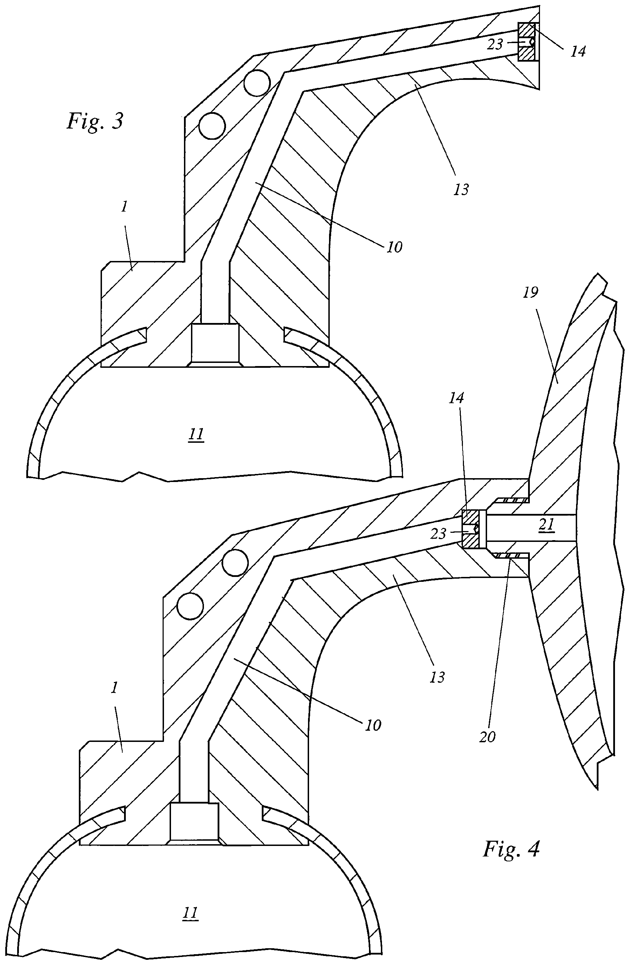

Die Sprühköpfe 1 in Fig. 3 und Fig. 4 sind ähnlich aufgebaut wie in Fig. 1 jedoch ohne den Sprühbehälter 2 abgebildet. Die Adapterhülle 11 ist jeweils nur teilweise dargestellt.

In Fig. 3 ist die Sprühkopfkulisse 13 des Sprühkopfs 1 als von den Lippen eines Patienten umschließbares Mundstück ausgeführt. Nahe an dessen Ende ist der Düsenkörper 14 eingesetzt.

In Fig. 4 weist die Sprühkopfkulisse 13 ein Gewinde 20 auf, über welches verschiedene Patientenluftwegeadapter 19 anschraubbar sind. Unterbrochen dargestellt ist ein als Mund-Nasen-Maske ausgebildeter Patientenluftwegeadapter 19, welcher aus einem festen oder vorzugsweise flexiblen Kunststoff, wie beispielsweise einem Silikonkautschuk, hergestellt sein kann, um Nase und Mund eines Patienten sicher zu umschließen. Ist die Maske selbst flexibel ausgeführt, empfiehlt sich, das Gegenstück zum Gewinde 20 der Sprühkopfkulisse dennoch aus einem festen Kunststoff auszuführen, d.h. den Patientenluftwegeadapter 19 aus zweierlei entsprechend geeigneten Materialien zusammenzufügen.

In der dargestellten Variante ist das Gewinde 20 als Innengewinde ausgeführt, so dass die Sprühkopfkulisse 13 als Mundstück dienen kann, wenn der Patientenluftwegeadapter 19 nicht aufgeschraubt ist.

Um einen breit aufgefächerten Sprühstrahl zu erhalten, empfiehlt es sich, den Sprühkanal 21, in welchen die Austrittsöffnungen 16a, 16b der Austrittskanäle 15a, 15b münden, möglichst kurz auszuführen. Ein längerer Sprühkanal 21 sorgt für einen weniger aufgefächerten Sprühstrahl, wobei sich allerdings eine größere Menge Sprühgut an der Wand des Sprühkanals niederschlägt.

Figuren 6a-d zeigen einen Düsenkörper 14 mit drei Paaren von Austrittskanälen 15a, 15b, 25a, 25b und 35a, 35b mit unterschiedlichen Kanaldurchmessern d₁, d₂, und d₃. Fig. 6a ist dabei die Draufsicht von unten in Figuren 6b-d. Die Schnittebene der Fig. 6b ist in Fig. 6a als durchbrochene Linie B-B' angedeutet, die Schnittebene der Fig. 6c als durchbrochene Linie C-C', und die Schnittebene der Fig. 6d als durchbrochene Linie D-D'. Die Stufen 24 und 30 ermöglichen verschiedene Längen l₁, l₂, und l₃ für die Austrittskanäle 15a, 15b, 25a, 25b bzw. 35a, 35b so dass sich das Aspektverhältnis für jedes Paar Austrittskanäle 15a, 15b, 25a, 25b bzw. 35a, 35b über die Wahl der jeweiligen Stegdicke jeweils gleich oder voneinander verschieden einstellen lässt. Der Strahl-Prallwinkel α in den Schnittpunkten S₁, S₂, und S₃ der Kanalmittelachsen 17a, 17b, 27a, 27b bzw. 37a, 37b kann ebenfalls jeweils unterschiedlich gewählt werden.

Durch die parallelen Austrittskanalpaare 15a, 15b, 25a, 25b bzw. 35a, 35b mit zugehörigen Austrittsöffnungen 16a, 16b, 26a, 26b, 36a, 36b lässt sich so eine breitere Tröpfchengrößenverteilung erzielen. Insbesondere voneinander abweichende Aspektverhältnisse sorgen für unterschiedlich starke Druckverluste der jeweiligen Sprühstrahle und somit unterschiedlich hohen Energieeintrag beim Aufprall im jeweiligen Schnittpunkt S₁, S₂, und S₃ der Kanalmittelachsen.

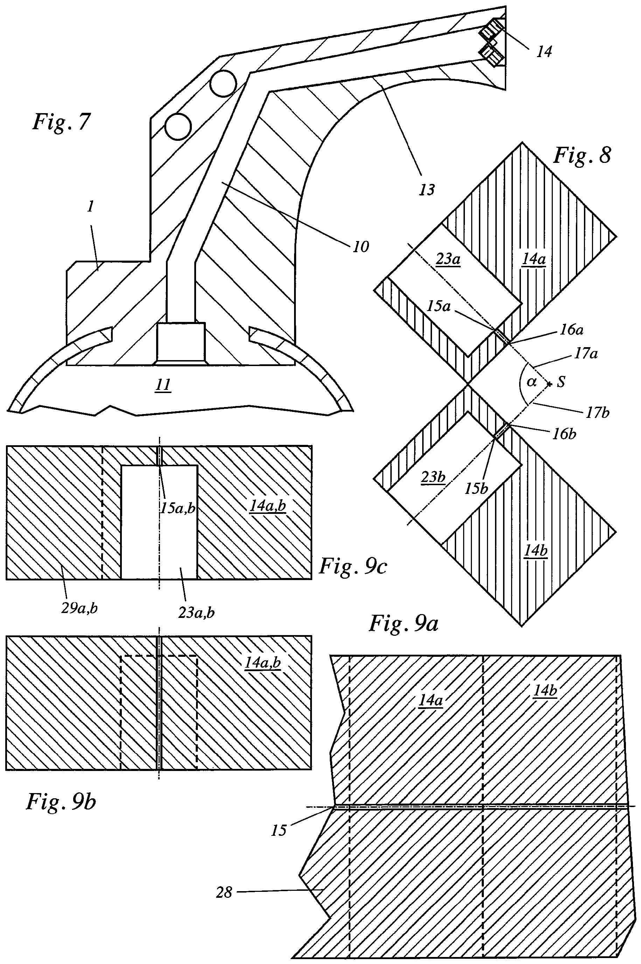

Fig. 7 zeigt die Querschnittsdarstellung eines erfindungsgemäßen, mit einem Mundstück integral geformten Sprühkopfs 1 ähnlich Fig. 3. Allerdings sind hier zwei Düsenkörper 14a, 14b mit jeweils einem Austrittskanal 15a, 15b vorgesehen. Die Anordnung der Düsenkörper 14a, 14b im Winkel α zueinander ist in Fig. 8 vergrößert dargestellt.

Figuren 9a-c illustrieren schematisch die Fertigung der Düsenkörper 14a, 14b. Die Glaskapillare 28 wird mit einem zylindrischen Kanal 15 im für die Austrittskanäle 15a, 15b gewählten Zylinderdurchmesser gezogen. Die Glaskapillare 28 wird in Scheiben 14a, 14b geschnitten. Der in Fig. 9b strichliert umrandete Bereich wird durch Einwirkung eines Picosekunden-Lasers abgetragen, um die jeweilige Fortsetzung 23a, 23b des Zuführungskanals 10 zu bilden. Ein Randbereich 29 a, b wird zudem durch Schleifen oder ebenfalls mittels Einwirkung eines Picosekunden-Lasers abgetrennt, um die gewünschte Einbaulage der Düsenkörper 14a, 14b in die Sprühkopfkulisse 13 zu ermöglichen.

Die vom Rand der Austrittsöffnungen 16a, 16b aufgespannten Austrittsquerschnittsflächen sind zueinander geneigt.

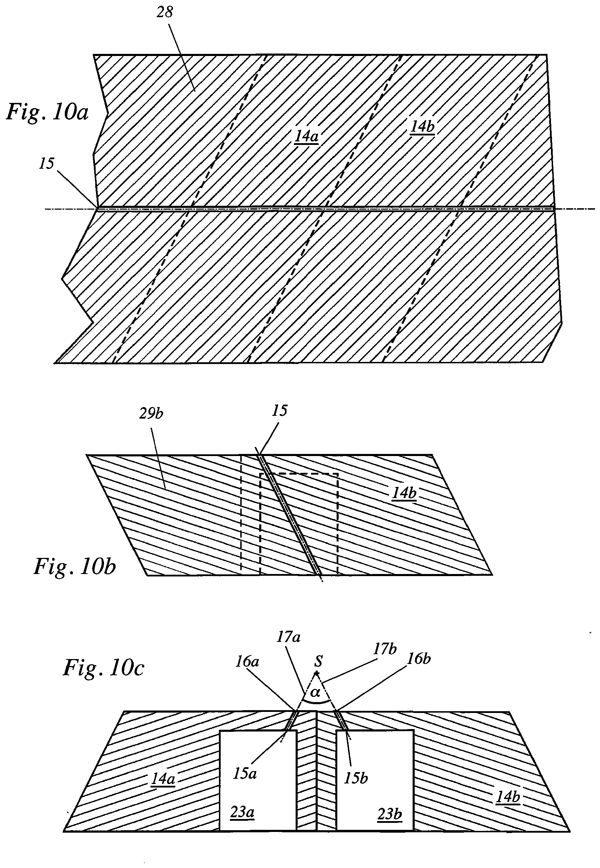

Figuren 10a-c illustrieren schematisch die Fertigung von Düsenkörpern 14a, 14b ähnlich Figuren 9a-c. Allerdings wird die gezogene Kapillare 15, wie durch die strichlierten Linien in Fig. 10a angedeutet, schräg geschnitten, um den Strahl-Prallwinkel α entsprechend einzustellen und zugleich die Stirnflächen der Scheiben 14a, 14b auf einer gemeinsamen Ebene (z.B. in einer gemeinsamen Montage-Nut) plan aufliegen zu lassen, wie in Fig. 10c angedeutet.

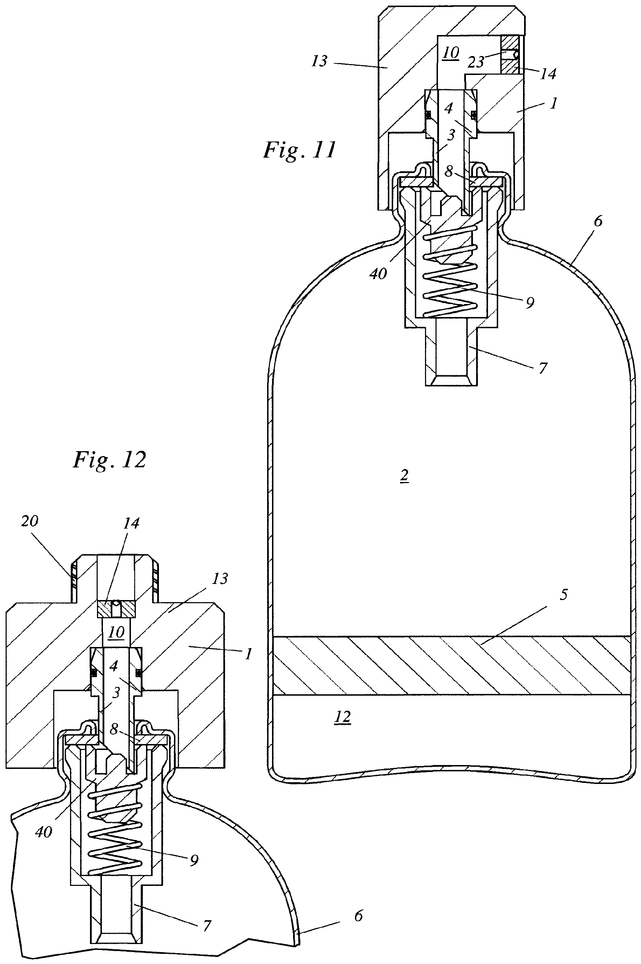

Figuren 11 und 12 zeigen erfindungsgemäße Sprühdosen im Querschnitt ähnlich Fig. 1 jedoch ohne Adapterhülle 11, wobei in Fig. 12 der Sprühbehälter 6 nur ausschnittweise dargestellt ist.

Die Sprühdose in Fig. 11 weist einen Sprühkopf 1 auf, in dessen Sprühkopfkulisse der Düsenkörper 14 so eingesetzt ist, dass der Sprühgutaustrag zur Seite erfolgt. Eine derarartige Anordnung ist für vielerlei technische (beispielsweise zur Desinfektion) oder kosmetische (z.B. zur Hautbefeuchtung oder zum Auftrag von Duftstoffen) Anwendungen vorteilhaft.

In Fig. 12 setzt sich die Sprühkopfkulisse in einem Stutzen mit Gewinde 20 fort. Für Inhalationsanwendungen kann hier ein geeigneter Patientenlftwegeadapter (in Fig. 12 nicht dargestellt) aufgeschraubt werden. Auch für anderweitige Adapter beispielsweise in technischen Anwendungen kann ein solcher Gewindestutzen vorteilhaft sein.

## Patentansprüche

1. Sprühkopf (1), welcher mindestens zwei Austrittskanäle (15a, 15b) aufweist, welche so angeordnet sind, dass aus Austrittsöffnungen (16a, 16b) jeweils mindestens zweier der Austrittskanäle austretende Sprühstrahle an einem von den Austrittsöffungen (16a, 16b) beabstandeten jeweiligen Aufprallort (S) zentral aufeinander treffen,
**dadurch gekennzeichnet, dass**
die Austrittskanäle (15a, 15b) zumindest über einen Teil ihrer Länge in Quarzglas ausgebildet sind,
die Austrittskanäle (15a, 15b) jeweils ein als Quotient aus Länge (l) des Austrittskanals und geringstem Durchmesser des Austrittskanals gebildetes Aspektverhältnis von 8 oder kleiner, vorzugsweise von 5 oder kleiner aufweisen, und
der jeweils geringste Durchmesser eines jeden Austrittskanals 50 µm oder kleiner ist.

2. Sprühkopf (1) gemäß Anspruch 1, wobei mindestens zwei der Austrittskanäle (15a, 15b) jeweils einen kleinsten Durchmesser zwischen 5 und 20 µm, vorzugsweise zwischen 5 und 15 µm, aufweisen.

3. Sprühkopf (1) gemäß einem der vorangehenden Ansprüche, wobei die Austrittskanäle (15a, 15b) jeweils eine Mittelachse (17a, 17b) aufweisen, welche zur jeweiligen Mittelachse eines jeweils anderen der Austrittskanäle in einem Winkel (α) zwischen 40 und 120 Grad, vorzugsweise zwischen 70 und 90 Grad, steht.

4. Sprühkopf (1) gemäß einem der vorangehenden Ansprüche, wobei die vom Rand der jeweiligen Austrittsöffnungen aufgespannten Austrittsquerschnittsflächen zueinander geneigt sind.

5. Sprühkopf (1) gemäß einem der vorangehenden Ansprüche, welcher mindestens zwei erste Austrittskanäle (15a, 15b) aufweist, welche so angeordnet sind, dass aus deren Austrittsöffnungen (16a, 16b) austretende Sprühstrahle an einem von den Austrittsöffungen beabstandeten ersten Aufprallort (S₁) zentral aufeinander treffen, und mindestens zwei zweite Austrittskanäle (25a, 25b), welche so angeordnet sind, dass aus deren Austrittsöffnungen (26a, 26b) austretende Sprühstrahle an einem von den Austrittsöffungen (26a, 26b) beabstandeten zweiten Aufprallort (S₂) zentral aufeinander treffen, wobei der geringste Austrittskanaldurchmesser der ersten Austrittskanäle vom geringsten Austrittskanaldurchmesser der zweiten Austrittskanäle abweicht und/oder das als Quotient aus Austrittskanallänge und geringstem Austrittskanaldurchmesser gebildete Aspektverhältnis der ersten Austrittskanäle vom als Quotient aus Austrittskanallänge und geringstem Austrittskanaldurchmesser gebildeten Aspektverhältnis der zweiten Austrittskanäle abweicht und/oder der jeweilige Winkel zwischen Mittelachsen der ersten Austrittskanäle vom jeweiligen Winkel zwischen Mittelachsen der zweiten Austrittskanäle abweicht.

6. Sprühkopf (1) gemäß einem der vorangehenden Ansprüche, wobei die Austrittskanäle (15a, 15b) jeweils vollständig in Quarzglas ausgebildet sind.

7. Sprühkopf (1) gemäß einem der Ansprüche 1 bis 5, wobei die Austrittskanäle (15a, 15b) jeweils in einem Abschnitt einer gezogenen Glaskapillare ausgebildet sind.

8. Sprühkopf (1) gemäß einem der vorangehenden Ansprüche, welcher mit einem Patientenluftwegeadapter (19) integral geformt oder verbunden ist, der als in eine Patientennase einführbarer Nasenadapter, als einen Patientenmund und/oder eine Patientennase abdeckender Maskenadapter oder mit einem Patientenmund umschließbarer Adapter ausgebildet ist.

9. Sprühdose, welche einen Sprühkopf (1) gemäß einem der Ansprüche 1-8 und einen Sprühbehälter (2) aufweist, der mit einer Flüssigkeit, vorzugsweise Sole, und einem Treibmittel befüllt ist.

10. Sprühdose gemäß Anspruch 9, wobei der Druck im Sprühbehälter (2) 3 MPa (30 bar), vorzugsweise 1,5 MPa (15 bar) nicht übersteigt.

11. Verfahren zum Herstellen eines Sprühkopfe (1), wobei zwei einander zugeneigte Austrittskanäle (15a, 15b) gebildet werden, **dadurch gekennzeichnet, dass**
die Austrittskanäle (15a, 15b) mit einem jeweiligen geringsten Durchmesser von 50 µm oder kleiner zumindest teilweise in Quarzglas so gebildet werden, dass die Austrittskanäle (15a, 15b) jeweils ein als Quotient aus Länge (l) des Austrittskanals und geringstem Durchmesser des Austrittskanals gebildetes Aspektverhältnis von 8 oder kleiner, vorzugsweise von 5 oder kleiner aufweisen.

12. Verfahren gemäß Anspruch 11, wobei zum Bilden der Austrittskanäle (15a, 15b) das Quarzglas lokal einer Lasereinwirkung ausgesetzt wird und anschließend das der Lasereinwirkung ausgesetzte Quarzglas weggeätzt wird.

13. Verfahren gemäß Anspruch 11, wobei die Austrittskanäle (15a, 15b) mittels Femtosekundenlaser gebohrt werden.

14. Verfahren gemäß Anspruch 11, wobei mindestens eine Glaskapillare (28) mit einem Innendurchmesser gezogen wird, der dem geringsten Durchmesser eines Austrittskanals entspricht, die mindestens eine Glaskapillare (28) so geschnitten wird, dass für jeden Austrittskanal (15a, 15b) ein diesen bildender Abschnitt entsteht, und die Abschnitte so zueinander angeordnet werden, dass die durch die Abschnitte gebildeten Austrittskanäle (15a, 15b) einander zugeneigt sind.

## Claims

1. A spray head (1) comprising at least two outlet channels (15a, 15b) arranged such that spray jets emerging from outlet orifices (16a, 16b) of at least two respective outlet channels meet centrally at a respective impact location spaced apart from the outlet orifices (16a, 16b),
**characterized in that**
the outlet channels (15a, 15b) are formed in quartz glass over at least part of their lengths,
the outlet channels (15a, 15b) each have an aspect ratio of 8 or less, preferably 5 or less, given as the quotient of the length (1) of the outlet channel and the smallest diameter of the outlet channel, and
the smallest diameter of each outlet channel is 50 µm or less.

2. The spray head (1) according to claim 1, wherein at least two of the outlet channels (15a, 15b) each have a respective smallest diameter between 5 and 20 µm, preferably between 5 and 15 µm.

3. The spray head (1) according to any one of the preceding claims, wherein the outlet channels (15a, 15b) each have a respective central axis (17a, 17b) which is at an angle (α) of between 40 and 120 degrees, preferably between 70 and 90 degrees, relative to the respective central axis of a respective other one of the outlet channels.

4. The spray head (1) according to any one of the preceding claims, wherein the outlet cross-sectional areas spanned by the edge of the respective outlet orifices are inclined relative to one another.

5. The spray head (1) according to any one of the preceding claims, which comprises at least two first outlet channels (15a, 15b) which are arranged such that spray jets emerging from their outlet orifices (16a, 16b) meet centrally at a first impact location (S₁) spaced apart from the outlet orifices, and at least two second outlet channels (25a, 25b) which are arranged such that spray jets emerging from their outlet orifices (26a, 26b) meet centrally at a second impact location (S₂) spaced apart from the outlet orifices (26a, 26b),
wherein the smallest outlet channel diameter of the first outlet channels deviates from the smallest outlet channel diameter of the second outlet channels and/or the aspect ratio of the first outlet channels formed as a quotient of outlet channel length and smallest outlet channel diameter deviates from the aspect ratio of the second outlet channels formed as a quotient of outlet channel length and smallest outlet channel diameter and/or the respective angle between central axes of the first outlet channels deviates from the respective angle between central axes of the second outlet channels.

6. The spray head (1) according to any one of the preceding claims, wherein the outlet channels (15a, 15b) are each completely formed of quartz glass.

7. The spray head (1) according to any one of claims 1 to 5, wherein the outlet channels (15a, 15b) are each formed in a section of a drawn glass capillary.

8. The spray head (1) according to any one of the preceding claims, which is integrally formed or connected to a patient airway adapter (19) which is formed as a nose adapter insertable into a patient nose, a mask adapter covering a patient mouth and/or patient nose, or an adapter enclosable with a patient mouth.

9. A spray can comprising a spray head (1) according to any one of claims 1-8 and a spray container (2) filled with a liquid, preferably brine, and a propellant.

10. The spray can according to claim 9, wherein the pressure in the spray container (2) does not exceed 3 MPa (30 bar), preferably 1,5 MPa (15 bar).

11. A method for manufacturing a spray head (1), wherein two outlet channels (15a, 15b) inclined towards each other are formed,
**characterised in that**
the outlet channels (15a, 15b) having a respective smallest diameter of 50 µm or smaller are at least partially formed in quartz glass such that the outlet channels (15a, 15b) each have an aspect ratio of 8 or smaller, preferably 5 or smaller, given as a quotient of the length of the outlet channel and the smallest diameter of the outlet channel.

12. The method according to claim 11, wherein, for forming the outlet channels (15a, 15b), the quartz glass is locally exposed to a laser beam and the quartz glass exposed to the laser beam is subsequently etched away.

13. The method according to claim 11, wherein the outlet channels (15a, 15b) are drilled using a femto-second laser.

14. The method according to claim 11, wherein at least one glass capillary (28) is drawn with an inner diameter corresponding to the smallest diameter of an outlet channel, the at least one glass capillary (28) is cut in such a way that for each outlet channel (15a, 15b) a section forming it is formed, and the sections are arranged relative to each other in such a way that the outlet channels (15a, 15b) formed by the sections are inclined towards each other.

## Revendications

1. Tête de pulvérisation (1), laquelle présente au moins deux canaux de sortie (15a, 15b), lesquels sont agencés de sorte que des jets de pulvérisation sortant des ouvertures de sortie (16a, 16b) de respectivement au moins deux des canaux de sortie se rencontrent centralement à un point d'impact (S) respectif espacé des ouvertures de sortie (16a, 16b),
**caractérisée en ce que**
les canaux de sortie (15a, 15b) sont réalisés au moins sur une partie de leur longueur en verre de quartz,
les canaux de sortie (15a, 15b) présentent respectivement un rapport d'aspect, formé en tant que quotient de la longueur (1) du canal de sortie et du plus petit diamètre du canal de sortie, de 8 ou moins, de préférence de 5 ou moins, et
le diamètre respectivement le plus petit de chaque canal de sortie est de 50 µm ou moins.

2. Tête de pulvérisation (1) selon la revendication 1, dans laquelle au moins deux des canaux de sortie (15a, 15b) présentent respectivement un diamètre le plus petit entre 5 et 20 µm, de préférence entre 5 et 15 µm.

3. Tête de pulvérisation (1) selon l'une quelconque des revendications précédentes, dans laquelle les canaux de sortie (15a, 15b) présentent respectivement un axe médian (17a, 17b), lequel forme un angle (α) entre 40 et 120 degrés, de préférence entre 70 et 90 degrés par rapport à l'axe médian respectif d'un autre respectivement des canaux de sortie.

4. Tête de pulvérisation (1) selon l'une quelconque des revendications précédentes, dans laquelle les sections transversales de sortie définies par le bord des ouvertures de sortie respectives sont inclinées l'une vers l'autre.

5. Tête de pulvérisation (1) selon l'une quelconque des revendications précédentes, laquelle présente au moins deux premiers canaux de sortie (15a, 15b), lesquels sont agencés de sorte que des jets de pulvérisation sortant de leurs ouvertures de sortie (16a, 16b) se rencontrent centralement à un premier point d'impact (S₁) espacé des ouvertures de sortie, et au moins deux seconds canaux de sortie (25a, 25b), lesquels sont agencés de sorte que des jets de pulvérisation sortant de leurs ouvertures de sortie (26a, 26b) se rencontrent centralement à un second point d'impact (S₂) espacé des ouvertures de sortie (26a, 26b),
dans laquelle le plus petit diamètre de canal de sortie des premiers canaux de sortie diverge du plus petit diamètre de canal de sortie des seconds canaux de sortie et/ou le rapport d'aspect formé en tant que quotient de la longueur de canal de sortie et du plus petit diamètre de canal de sortie des premiers canaux de sortie diverge du rapport d'aspect formé en tant que quotient de la longueur de canal de sortie et du plus petit diamètre de canal de sortie des seconds canaux de sortie et/ou l'angle respectif entre des axes médians des premiers canaux de sortie diverge de l'angle respectif entre des axes médians des seconds canaux de sortie.

6. Tête de pulvérisation (1) selon l'une quelconque des revendications précédentes, dans laquelle les canaux de sortie (15a, 15b) sont réalisés respectivement complètement en verre de quartz.

7. Tête de pulvérisation (1) selon l'une quelconque des revendications 1 à 5, dans laquelle les canaux de sortie (15a, 15b) sont réalisés respectivement dans une section d'un capillaire de verre tiré.

8. Tête de pulvérisation (1) selon l'une quelconque des revendications précédentes, laquelle est formée ou reliée d'un seul tenant avec un adaptateur des voies respiratoires d'un patient (19), qui est réalisé en tant qu'adaptateur nasal pouvant être introduit dans le nez d'un patient, en tant qu'adaptateur de masque recouvrant la bouche d'un patient et/ou le nez d'un patient ou avec un adaptateur entourant la bouche d'un patient.

9. Boîte de pulvérisation, laquelle présente une tête de pulvérisation (1) selon l'une quelconque des revendications 1 à 8 et un récipient de pulvérisation (2), qui est rempli d'un liquide, de préférence de saumure, et d'un agent propulseur.

10. Boîte de pulvérisation selon la revendication 9, dans laquelle la pression dans le récipient de pulvérisation (2) ne dépasse pas 3 MPa (30 bar), de préférence 1,5 MPa (15 bar).

11. Procédé de fabrication d'une tête de pulvérisation (1), dans lequel deux canaux de sortie (15a, 15b) inclinés l'un vers l'autre sont formés,
**caractérisé en ce que**
les canaux de sortie (15a, 15b) avec un diamètre le plus petit respectif de 50 µm ou moins sont formés au moins en partie en verre de quartz, de sorte que les canaux de sortie (15a, 15b) présentent respectivement un rapport d'aspect formé en tant que quotient de la longueur (1) du canal de sortie et du plus petit diamètre du canal de sortie de 8 ou moins, de préférence de 5 ou moins.

12. Procédé selon la revendication 11, dans lequel pour la formation des canaux de sortie (15a, 15b), le verre de quartz est soumis localement à un effet laser et ensuite le verre de quartz soumis à l'effet laser est décapé.

13. Procédé selon la revendication 11, dans lequel les canaux de sortie (15a, 15b) sont alésés au moyen d'un laser femtoseconde.

14. Procédé selon la revendication 11, dans lequel au moins un capillaire de verre (28) est tiré avec un diamètre intérieur, qui correspond au plus petit diamètre d'un canal de sortie, l'au moins un capillaire de verre (28) est coupé de sorte que pour chaque canal de sortie (15a, 15b), une section formant celui-ci est générée, et les sections sont agencées l'une par rapport à l'autre de sorte que les canaux de sortie (15a, 15b) formés par les sections sont inclinés l'un vers l'autre.
